# EUROPEAN PATENT APPLICATION

(11) **EP 1 894 561 A1**
(43) Date of publication of application: **05.03.2008**
(21) Application number: 07016934.7
(22) Date of filing: 29.08.2007
(51) Int. Cl.: A61K 9/50, A61K 31/519

(54) **Dipyridamole pharmaceutical compositions**

(30) Priority: 30.08.2006 US 823978 P
(71) Applicant: Dr. Reddy's Laboratories Ltd., Hyderabad 500 016 (IN); Dr. Reddy`s Laboratories Inc., Bridgewater NJ 08807-2862 (US)
(72) Inventor: Patil, Atul Vishvanath, Ameerpet Hyderabad A.P. (IN); Mohanty, Bishnu Bibha, Hyderabad A.P. (IN); Ravinder, Kodipyaka, Kukatpally Hyderabad 500 072, A.P. (IN); Bakshi, Gautam, Miyapur Hyderabad 500 050, A.P. (IN); Singh, Gurvinder, Secunderabad 500 009, A.P. (IN); Bhushan, Indu, Hyderabad 500 072, A.P. (IN); Mohan, Mailatur Sivaraman, Kukatpally Hyderabat 500 072, A.P. (IN)
(74) Representative: Müller-Boré & Partner Patentanwälte

(57) **Abstract**

Pharmaceutical compositions comprise a pharmaceutically inert core coated with an intermediate coating, a coating thereon comprising dipyridamole, and a further coating comprising a drug release modifier.

## Description

The present invention relates to pharmaceutical compositions comprising dipyridamole, and process to prepare such compositions.

Dipyridamole has a chemical name 2,2',2",2"'-[(4,8-Dipiperidinopyrimido [5,4-*d*] pyrimidine-2,6-diyl)dinitrilo]-tetraethanol and is represented by structural Formula I. It is commercially available in the form of oral tablets of 25, 50 and 75 mg strengths under the brand name PERSANTINE™, and as extended release capsules of 200 mg strength under the brand name PERSANTIN RETARD™. It is also available as a combination product of aspirin and dipyridamole (25 mg/ 200 mg) in the form of extended release capsules under the brand name AGGRENOX™. Boehringer Ingelheim manufactures these products.

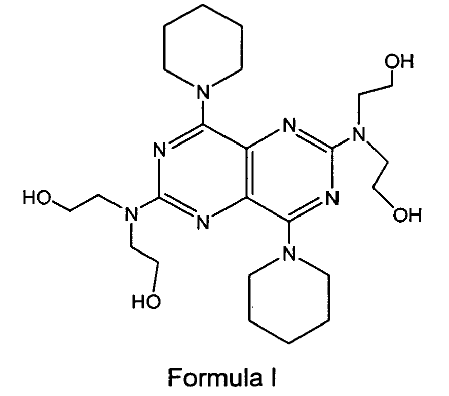

U.S. Patent No. 4,361,546 describes a gelatin capsule dosage form that contains an easily disintegrating tablet comprising dipyridamole and having a water-soluble coating, plus five "retard" tablets, each containing a mixture of dipyridamole and tartaric acid and further having an insoluble lacquer coating.

U.S. Patent No. 4,367,217 describes a sustained release composition comprising spheroidal particles prepared by granulating a mixture of dipyridamole and an organic acid, and coating with an acid-insoluble lacquer coating.

U.S. Patent No. 6,015,577 describe pharmaceutical combinations of dipyridamole or mopidamol with acetylsalicylic acid.

While preparing compositions similar to those disclosed in U.S. Patent Nos. 4,361,546 and 4,367,217, it was observed that high amounts of impurities are generated. Without being bound by any theory, this was ascribed to an incompatibility between dipyridamole and acidic excipients which is believed to generate high levels of impurities.

Thus, development of pharmaceutical compositions of dipyridamole as described in the context of the present invention would be a significant improvement in the field of pharmaceutical technology.

The present invention relates to pharmaceutical compositions of dipyridamole.

An aspect of the present invention relates to stable pharmaceutical compositions comprising:
a core having an intermediate coating comprising a hydrocolloid;
a dipyridamole layer on top of the intermediate layer; and
an outermost layer comprising a drug release modifying substance.

In an embodiment, the cores of the present invention comprise tartaric acid, citric acid, ascorbic acid, malic acid, succinic acid or mixtures thereof.

In another embodiment, an intermediate coating that prevents direct contact between dipyridamole and the core comprises hydrophilic or hydrophobic materials, or a combination thereof.

In a further embodiment of the present invention, the intermediate coating comprises one or more sub-coatings of different hydrophilic or hydrophobic materials, or a combination thereof.

A still further embodiment of the present invention provides stable pharmaceutical compositions comprising a core having an intermediate coating comprising a hydrocolloid, and a layer comprising dipyridamole and a drug release modifying substance on top of said intermediate layer.

Another aspect of the present invention provides for a process for preparing stable pharmaceutical compositions comprising:
a core having an intermediate coating comprising a hydrocolloid;
a dipyridamole layer on top of the intermediate layer; and
an outermost layer comprising a drug release modifying substance.

The present invention relates to pharmaceutical compositions of dipyridamole.

The present invention also relates to pharmaceutical compositions of dipyridamole, which comprise at least one core having an intermediate coating, a dipyridamole layer, and a drug release-modifying layer.

The present invention also relates to pharmaceutical compositions of dipyridamole, which comprise at least one core having an intermediate coating and a second layer comprising dipyridamole and a drug release modifying substance.

In the context of the present invention, the terms "cores" or "beads" or "spheres" or "granules" or "pellet" or "inert particles" or "particles" or "nucleus" are used synonymously.

Further in the context of the present invention, the terms "layer" or "coating" or "deposit" is used synonymously.

An embodiment of the present invention relates to stable pharmaceutical compositions comprising:
a core having an intermediate coating comprising a hydrocolloid;
a dipyridamole layer on top of the intermediate layer; and
an outermost layer comprising a drug release modifying substance.

In an embodiment, the cores of the present invention comprise tartaric acid, citric acid, ascorbic acid, malic acid, succinic acid or mixtures thereof.

In another embodiment, an intermediate coating that prevents direct contact between dipyridamole and the core comprises a hydrocolloid, optionally with other hydrophilic or hydrophobic materials, or a combination thereof. An intermediate coating can be formed from more than one separate layer of pharmaceutically acceptable excipients.

In a further embodiment of the present invention, the intermediate coating comprises one or more layers of different hydrophilic or hydrophobic materials, or a combination thereof.

A still further embodiment of the present invention provides stable pharmaceutical compositions comprising a core having an intermediate coating comprising a hydrocolloid, and a layer comprising dipyridamole and a drug release modifying substance on top of said intermediate layer.

The cores found useful in the context of present invention include but are not limited to: water-soluble cores such as sucrose spheres, lactose, organic acids such as tartaric acid, citric acid, ascorbic acid, malic acid, succinic acid, and the like; and water-insoluble cores such as microcrystalline cellulose, silicon dioxide, calcium carbonate, dicalcium phosphate anhydrous, dicalcium phosphate monohydrate, tribasic calcium phosphate, magnesium carbonate, magnesium oxide, and the like and mixtures thereof. In an embodiment, tartaric acid cores have been found to be useful.

As described earlier, and without being bound by any particular theory of operation, direct contact between dipyridamole and tartaric acid is believed to generate high levels of tartaric acid adduct impurity as a degradation product. A tartaric acid adduct impurity has a chemical name 2,3-Dihydroxy-succinic acid mono{2-[{6-[bis-(2-hydroxy-ethyl)-amino]-4-piperidin-1-yl-pyrimido[5,4-d]pyrimidin-2-yl}-(2-hydroxy-ethyl)-amino}-ethyl] ester, and is represented by structural Formula 2.

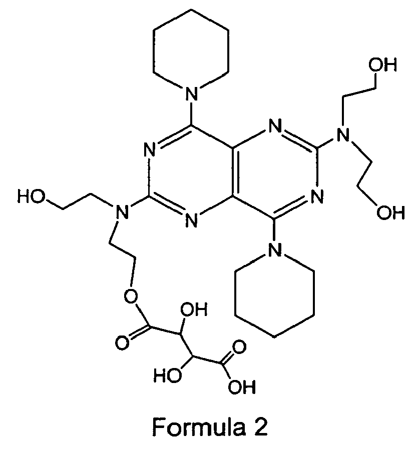

The tartaric acid adduct impurity can be analyzed in dipyridamole compositions using the following high performance liquid chromatography analytical procedure:
Buffer Preparation:
Potassium dihydrogen phosphate (1.36 g) was dissolved in 1 L of MilliQ water with sonication, followed by adjusting pH to 3 using orthophosphoric acid solution and filtration through a 0.45 µm Durapore PVDF hydrophilic membrane filter.
Mobile phase A:
Buffer preparation and acetonitrile were mixed in the ratio of 90:10 v/v followed by degassing in a sonicator for 10 minutes.
Mobile phase B:
Buffer preparation and acetonitrile were mixed in the ratio of 30:70 v/v followed by degassing in a sonicator for 10 minutes.
With the above mobile phases, a gradient program of 0, 20, 35, 37, and 45 minutes is performed with varying compositions-of mobile phase for each time period, as given in the table below:

| **TIme (minutes)** | **% of Mobile Phase A** | **% of Mobile Phase B** |
|---|---|---|
| 0 | 100 | 0 |
| 20 | 50 | 50 |
| 35 | 0 | 100 |
| 37 | 100 | 0 |
| 45 | 100 | 0 |

Chromatographic system:
Liquid chromatograph equipped with UV visible detector at 290 nm.
Column: ACE-C8, 250 mm x 4.6 mm, 5 µm, or equivalent.
Column temperature: Ambient.
Flow rate: 1.5 mL per minute.
Injection volume: 10 µL.
Limit of detection: 0.004%.
Limit of Quantification (LOQ): 0.012%.
Relative Retention Time (RRT): 0.92, when dipyridamole is assigned a retention time of 1.
Relative Response Factor (RRF): 0.80

Other impurities that can be generated in dipyridamole pharmaceutical formulations include "Impurity A', 'Impurity B', Impurity C,' as described in the *British Pharmacopeia* monograph for dipyridamole.

Impurity A has a chemical name 2,2'-[[4,6,8-tri(piperidin-1-yl)pyrimido[5,4-d]pyrimidin-2-yl]nitrilo]diethanol, and structural Formula 3.
Relative Retention Time (RRT): 1.84, where dipyridamole = 1.
Relative Response Factor (RRF): 1.10.

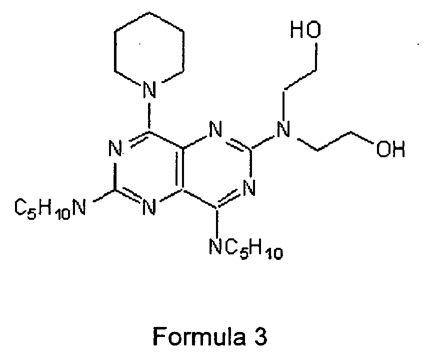

Impurity B has a chemical name 2,2', 2", 2"', 2"", 2""'-[[8-(piperidin-1-yl)pyrimido[5,4-d]pyrimidine-2,4,6-triyl]trinitrilo]hexaethanol, and structural Formula-4.
Relative Retention Time (RRT): 0.64, where dipyridamole = 1.
Relative Response Factor (RRF): 0.92.

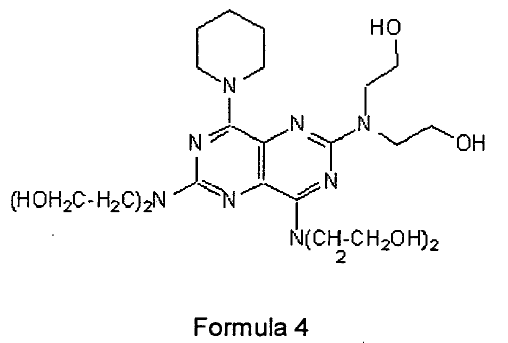

Impurity C has a chemical name 2,2'-[[2-chloro-4,8-di(piperidin-1-yl)pyrimido[5,4-d]pyrimidin-6-yl]nitrilo]diethanol, and structural Formula 5.

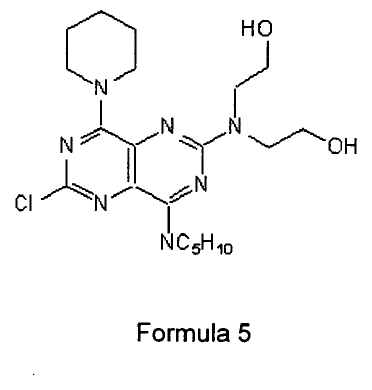

The term "hydrocolloid" in the context of the present invention refers to hydrophilic colloidal substances such as acacia gum (also known as gum Arabic), tragacanth, xanthan gum, carboxymethyl cellulose sodium, hydroxyethyl cellulose, hydroxypropyl methylcellulose ("HPMC"), hydroxypropyl cellulose, povidone, polyvinyl acetate ("PVA"), chitosan, povidone, and the like and combinations thereof.

Various materials that may be used in an intermediate coating include but are not limited to: hydrophilic materials such as homopolymers or copolymers of N-vinylpyrrolidone, cellulose derivatives such as hydroxypropyl methyl cellulose, vinyl and acrylic polymers; polyacrylic acid and the like; hydrophobic substances such as celluloses like ethyl cellulose, low substituted hydroxylpropyl cellulose ("L-HPC"), cellulose acetate, cellulose propionate (lower, medium or higher molecular weight), cellulose acetate propionate, cellulose acetate butyrate, cellulose acetate phthalate; polyalkyl methacrylates; polyalkyl acrylates; crosslinked vinylpyrrolidone polymers; hydrogenated castor oil; and the like. Other classes of rate controlling substances or their mixtures in various ratios as required are also within the purview of this invention without limitation.

The intermediate layer (or seal coating) may be coated over the cores by techniques such as powder coating, spray coating, dip coating, fluidized bed coating and the like.

In an embodiment, a core is coated with an intermediate coating and then layered with a dipyridamole coating. In an aspect of this embodiment, more than one intermediate coating between the core and the dipyridamole layer are also contemplated within the scope of the present invention.

In another embodiment, a pharmaceutically inert core is sprayed or layered over with a solution of organic acid(s) in a suitable solvent or solvent system, coated with an intermediate coating, and then layered with a dipyridamole coating.

Another aspect of the present invention provides for a process for preparing a stable pharmaceutical compositions comprising
a core having an intermediate coating comprising a hydrocolloid;
a dipyridamole layer on top of the intermediate layer; and
an outermost layer comprising a drug release modifying substance.

Further in an embodiment, pharmaceutical compositions of dipyridamole are prepared as follows:
a) Cores are layered or sprayed over with an intermediate layer, optionally with other pharmaceutically acceptable excipients, by techniques such as powder coating, spray coating, dip coating, fluidized bed coating, and the like.
b) The coated cores are layered with dipyridamole coat, optionally with other pharmaceutically acceptable excipients.
c) The cores formed in step b) are coated with drug release modifying substances such as cellulose polymers, cellulose phthalate polymers or their derivatives, methacrylic acid alkyl esters or its copolymers, and the like or mixtures thereof.
d) The cores of step c) are dried, and are filled into capsules or compressed into tablets, optionally with other pharmaceutically acceptable excipients.

In another embodiment of the present invention, seal coated cores can be layered or coated with dipyridamole by:
a) layering dipyridamole as a powder, along with solvent system optionally comprising a binder; or
b) layering dipyridamole as a suspension or solution with or without a binder in equipment such as a fluid bed processor.

In the context of the present invention, during the preparation of the pharmaceutical compositions of dipyridamole into finished dosage form, one or more pharmaceutically acceptable excipients may optionally be used, including but are not limited to: diluents such as microcrystalline cellulose (MCC), silicified MCC (e.g. Prosolv™ HD 90), microfine cellulose, lactose, starch, pregelatinized starch, mannitol, sorbitol, dextrates, dextrin, maltodextrin, dextrose, calcium carbonate, calcium sulfate, dibasic calcium phosphate dihydrate, tribasic calcium phosphate, magnesium carbonate, magnesium oxide and the like; binders such as acacia, guar gum, alginic acid, dextrin, maltodextrin, methylcellulose, ethyl cellulose, hydroxyethyl cellulose, hydroxypropyl cellulose (e.g. KLUCEL^{®}), hydroxypropyl methylcellulose (e.g. METHOCEL^{®}), carboxymethyl cellulose sodium, povidone (various grades of KOLLIDON^{®}, PLASDONE^{®}), starch and the like; disintegrants such as carboxymethyl cellulose sodium (e.g. Ac-Di-Sol^{®}, Primellose^{®}), crospovidone (e.g. Kollidon^{®}, Polyplasdone^{®}), povidone K-30, polacrilin potassium, starch, pregelatinized starch, sodium starch glycolate (e.g. Explotab^{®}) and the like; surfactants including anionic surfactants such as chenodeoxycholic acid, 1-octanesulfonic acid sodium salt, sodium deoxycholate, glycodeoxycholic acid sodium salt, N-lauroylsarcosine sodium salt, lithium dodecyl sulfate, sodium cholate hydrate, sodium lauryl sulfate (SLS) and sodium dodecyl sulfate (SDS); cationic surfactants such as cetylpyridinium chloride monohydrate and hexadecyl- trimethylammonium bromide; nonionic surfactants such as N-decanoyl-N-methylglucamine, octyl a-D-glucopyranoside, n-Dodecyl b-D-maltoside (DDM), polyoxyethylene sorbitan esters like polysorbates and the like; plasticizers such as acetyltributyl citrate, phosphate esters, phthalate esters, amides, mineral oils, fatty acids and esters, glycerin, triacetin or sugars, fatty alcohols, polyethylene glycol, ethers of polyethylene glycol, fatty alcohols such as cetostearyl alcohol, cetyl alcohol, stearyl alcohol, oleyl alcohol, myristyl alcohol and the like; solvents that may be used in granulation or layering or coating are such as aqueous like water or alcoholic like ethanol, isopropanolol or hydroalcoholic like a mixture of water with alcohol in any ratio or organic like acetone, methylene chloride, dichloromethane and the like.

Pharmaceutical compositions of the present invention may further include any one or more of pharmaceutically acceptable glidants, lubricants, opacifiers, colorants and other commonly used excipients.

Various pharmaceutically acceptable excipients that may be used as drug release modifying substances include but are not limited to cellulosic polymers such as hydroxypropylmethyl cellulose phthalate, hydroxypropylcellulose phthalate, hydroxypropyl methylcellulose hexahydrophthalate, cellulose acetate phthalate, cellulose ester-ether phthalate, alkali salts of cellulose acetate phthalate, alkaline earth salts of cellulose acetate phthalate, cellulose acetate hexahydrophthalate, acrylic acid polymers and copolymers such as methacrylic acid, acrylic acid alkyl esters, methacrylic acid alkyl esters; copolymers of acrylic acid, methacrylic acid, methyl acrylate, ethyl acrylate, methyl methacrylate and/or ethyl methacrylate with a terpolymer of ethyl acrylate, methyl methacrylate and trimethylammonioethyl methacrylate chloride (Eudragit^{®} RS); vinyl polymers and copolymers such as polyvinyl acetate, polyvinylacetate phthalate, vinylacetate crotonic acid copolymer and ethylene-vinyl acetate copolymers; shellac, ammoniated shellac, shellac-acetyl alcohol and shellac n-butyl stearate and the like. Other classes of polymers, copolymers of these polymers or their mixtures in various ratios as required are within the scope of this invention without limitation.

Many of the drug release modifying substances are generally insoluble in acidic environments such as gastric fluids, but are soluble in higher-pH environments such as the duodenum and lower areas of the gastrointestinal tract. These "enteric polymers" thus delay release of the drug substance contained inside a dosage form, by preventing contact of the drug and fluids until a higher-pH condition is encountered. By selecting appropriate polymers, drug release from a dosage form can be obtained in a desired part of the digestive system.

The compositions of the present invention may also be used with other anti-platelet actives, including but not limited to aspirin, clopidogrel and the like. Also the pharmaceutical compositions comprising dipyridamole particles of present invention and other active pharmaceutical ingredients are well within the purview of this invention. Such other active(s) can either be crystalline or amorphous in form or mixtures thereof, optionally with other pharmaceutically acceptable excipients.

The pharmaceutical compositions as disclosed in the context of the present invention are useful in the treatment or prophylaxis of thrombotic disorders in mammals such as humans.

The following examples will further illustrate certain specific aspects and embodiments of the invention in greater detail and are not intended to limit the scope of the invention.

### EXAMPLE 1: Dipyridamole 200 mg extended release capsules.

| **Ingredient** | **Grams/Batch#** |
|---|---|
| Tartaric acid pellets** | 634.8 |

| SEAL COATING: Stage 1 | |
|---|---|
| Hypromellose 15 cps^{$} | 71.8 |
| Isopropyl alcohol* | 1160 |
| Methylene chloride* | 1160 |

| SEAL COATING: Stage 2 | |
|---|---|
| Acacia | 71.8 |
| Water* | 1124 |

| SEAL COATING: Stage 3 | |
|---|---|
| Stearic acid | 11 |
| Isopropyl alcohol* | 127 |

| DRUG LAYERING | |
|---|---|
| Dipyridamole | 552 |
| Povidone K-30*** | 19.3 |
| Hypromellose 5 cps | 8.3 |
| Isopropyl alcohol* | 220 |
| Methylene chloride* | 146.6 |

| MODIFIED RELEASE COATING^{##} | |
|---|---|
| Eudragit S100^{$$} | 7.1 |
| Hypromellose phthalate 55 | 3.4 |
| Hypromellose 5 cps | 4 |
| Talc | 7.8 |
| Dimethicone | 0.1 |
| Triacetin | 1.7 |
| Stearic acid | 0.2 |
| Isopropyl alcohol* | 253 |
| Acetone* | 253 |
| **Total fill weight per capsule** | **538 mg** |

| | |
|---|---|
| * Evaporate during processing ** Composition: tartaric acid (96% w/w); sucrose (2% w/w), povidone K 30 (2% w/w). Supplied by Meenaxi Pharma India. Pellets are manufactured by wetting sucrose with an aqueous povidone dispersion in a coating pan and then adding tartaric acid and rolling the pan to get pellets, with continued povidone dispersion spraying. The rounded pellets are dried for freedom from sticking). *** BASF manufactures polyvinyl pyrrolidone (Povidone K-30). ^{$} Dow Chemical Company manufactures Hypromellose 5 cps. ^{$$} Rohm Gmbh & Company manufactures Eudragit S100. ^{#} Batch size 2760 capsules up to modified release coating. ^{##} Batch size 500 capsules for modified release coating. | |

### Manufacturing Process:

1. Hypromellose was dissolved in the mixture of isopropyl alcohol and methylene chloride. Tartaric acid pellets were coated with hypromellose solution to achieve a weight build up of 10-12%, in a fluid bed processor.
2. Acacia was dissolved in water. Hypromellose coated pellets of step 1 were coated with acacia solution to achieve a weight build up of 10-1-2%, in a fluid bed processor.
3. Stearic acid was dissolved in isopropyl alcohol. Acacia-coated pellets of step 2 were further coated with stearic acid solution to achieve a weight build up of 1-2%, in a fluid bed processor.
4. Povidone K-30 and hypromellose were dissolved in the mixture of isopropyl alcohol and methylene chloride. Pellets of step 3 were coated with dipyridamole using a powder layering technique with the Povidone K-30 and hypromellose binder solution.
5. Eudragit S 100, hypromellose phthalate, hypromellose, talc, stearic acid, dimethicone and triacetin were dispersed in the mixture of isopropyl alcohol and acetone.
6. Drug loaded pellets of step 4 were coated with the modified release coating suspension of step 5 to achieve a weight build up of 6-10%.
7. Modified release coated pellets (of step 6) equivalent to 200 mg of dipyridamole were filled into a hard gelatin capsule.

### In vitro dissolution testing data.

Procedure: Test 711 "Dissolution" from United States Pharmacopoeia 29, United States Pharmacopoeial Convention, Inc., Rockville, Maryland., 2005 ("USP").
Medium: 0.1 N HCI for 1 hour followed by pH 5.5 phosphate buffer.
Apparatus: USP type I (basket).
Volume: 900 ml.
Rotation speed: 100 rpm.

| **Time** **(Hours)** | **Cumulative % Drug Dissolved** | |
|---|---|---|
| | **EXAMPLE 1** | **PERSANTIN RETARD™** |
| 0 | 0 | 0 |
| 0.5 | 4 | 9 |
| 1 | 14 | 25 |
| 2 | 40 | 50 |
| 5 | 73 | 78 |
| 7 | 87 | 88 |
| 12 | 97 | 93 |

### EXAMPLES 2-3: Dipyridamole 200 mg extended release capsules with different seal coating compositions.

| **Ingredient** | **Grams/Batch** | |
|---|---|---|
| | **Example 2** | **Example 3** |
| Tartaric acid pellets | 501.4 | 634.8 |

| SEAL COATING: Stage 1 | | |
|---|---|---|
| Hypromellose 15 cps | 43.6 | 71.8 |
| Isopropyl alcohol* | 705 | 1160 |
| Methylene chloride* | 705 | 1160 |

| SEAL COATING: Stage 2 | | |
|---|---|---|
| Acacia | 56.7 | 71.8 |
| Water* | 888 | 1124 |

| SEAL COATING: Stage 3 | | |
|---|---|---|
| Stearic acid | 8.7 | 11.04 |
| Isopropyl alcohol* | 100 | 127 |

| DRUG LAYERING | | |
|---|---|---|
| Dipyridamole | 436 | 552 |
| Povidone K-30 | 10.7 | 19.3 |
| Hypromellose 5 cps | 4.6 | 8.3 |
| Isopropyl alcohol* | 203 | 220 |
| Methylene chloride* | 87 | 146.6 |

| MODIFIED RELEASE COATING^{#} | | |
|---|---|---|
| Eudragit S100 | 4.7 | 6.5 |
| Hypromellose phthalate 55 | 1.8 | 2.3 |
| Hypromellose 5 cps | 3.1 | 3.3 |
| Talc | 3.2 | 4 |
| Dimethicone | 0.04 | 0.05 |
| Triacetin | 1.1 | 1.3 |
| Stearic acid | 0.1 | 0.2 |
| Isopropyl alcohol* | 192 | 210 |
| Acetone* | 192 | 210 |
| **Total fill weight per capsule** | **530 mg** | **531 mg** |

| | | |
|---|---|---|
| * Evaporate during processing ^{#} Batch size = 410 capsules for modified release coating. | | |

Manufacturing process was similar to that described in Example 1.

### EXAMPLE 4: Dipyridamole 200 mg extended release capsules with a 1:1 weight ratio of acacia to hypromellose in seal coating stage 2.

| **Ingredient** | **Grams/Batch** |
|---|---|
| Tartaric acid pellets | 583 |

| SEAL COATING: Stage 1 | |
|---|---|
| Hypromellose 15 cps | 50.4 |
| Isopropyl alcohol* | 814 |
| Methylene chloride* | 814 |

| SEAL COATING: Stage 2 | |
|---|---|
| Acacia | 41.6 |
| Hypromellose 15 cps | 41.6 |
| Triacetin | 9.54 |
| Water* | 1453 |

| SEAL COATING: Stage 3 | |
|---|---|
| Stearic acid | 10.6 |
| Isopropyl alcohol* | 122 |

| DRUG LAYERING | |
|---|---|
| Dipyridamole | 265 |
| Povidone K-30 | 13 |
| Hypromellose 5 cps | 5.6 |
| Isopropyl alcohol* | 203 |
| Methylene chloride* | 87 |

| MODIFIED RELEASE COATING | |
|---|---|
| Eudragit S100 | 21.1 |
| Hypromellose phthalate 55 | 9.9 |
| Hypromellose 5 cps | 11.7 |
| Talc | 13.9 |
| Dimethicone | 0.1 |
| Triacetin | 5. |
| Stearic acid | 0.7 |
| Acetone* | 748.5 |
| Isopropyl alcohol | 748.5 |
| **Total fill weight per capsule** | **539 mg** |

| | |
|---|---|
| * Evaporate during processing | |

Manufacturing process was similar to that described in Example 1.

### EXAMPLE 5: Dipyridamole 200 mg extended release capsules.

| **Ingredient** | **Quantity** | |
|---|---|---|
| | **(% w/w)** | **mg/Capsule** |
| Tartaric acid pellets | 41-45 | 200-230 |

| SEAL COATING: Stage 1 | | |
|---|---|---|
| Hypromellose 15 cps | 3.1-5.6 | 10-30 |

| SEAL COATING: Stage 2 | | |
|---|---|---|
| Acacia | 3.1-5.6 | 15-30 |
| Hypromellose 5 cps | 0-5.5 | 0-30 |
| Triacetin | 0-0.5 | 0.3-4 |

| SEAL COATING: Stage 3 | | |
|---|---|---|
| Stearic acid | 0.7-0.9 | 0-4 |

| DRUG LAYERING | | |
|---|---|---|
| Dipyridamole | 37-42 | 200 |
| Povidone K-30 | 1.3-1.5 | 4-10 |
| Hypromellose 5 cps | 1.3-1.5 | 4-10 |

| MODIFIED RELEASE COATING | | |
|---|---|---|
| Eudragit S100 | 2-3 | 10-20 |
| Hypromellose phthalate 55 | 0.8-1.0 | 5-10 |
| Hypromellose 5 cps | 1-2 | 5-10 |
| Talc | 0.4-2.5 | 2-12 |
| Dimethicone | 0.002-0.004 | 0.05-0.2 |
| Triacetin | 0.4-0.6 | 2-4 |
| Stearic acid | 0.06-0.07 | 0.2-0.5 |
| **Average fill weight per capsule** | **-** | **480-550 mg** |

Manufacturing process is similar to that described in Example 1.

### COMPARATIVE EXAMPLE A: Dipyridamole 200 mg extended release capsules prepared by granulation and without seal coating.

| **Ingredient** | **Grams/Batch** |
|---|---|
| Tartaric acid | 1200 |
| Dipyridamole | 1200 |
| Povidone K-30 | 120 |
| Isopropyl alcohol* | 1080 |
| Eudragit S100 | 30 |
| Hypromellose 5 cps | 30 |
| Hypromellose phthalate 55 | 18 |
| Dimethicone | 4 |
| Triacetin | 12 |
| Isopropyl alcohol* | 522 |
| Acetone* | 522 |

| | |
|---|---|
| * Evaporate during processing | |

Manufacturing process
1. Povidone was dissolved in isopropyl alcohol.
2. Tartaric acid (240 g) was mixed with dipyridamole.
3. The mixture of step 2 was loaded onto the remaining tartaric acid (960 g) by a powder layering technique using binder solution of step 1.
4. The drug loaded particles of step 3 were coated with a modified release dispersion containing Eudragit, hypromellose phthalate, hypromellose, dimethicone and triacetin, dispersed in the mixture of isopropyl alcohol and acetone to a weight build up of 7.5% w/w, in a fluid bed processor.
5. Modified release coated particles equivalent to 200 mg dipyridamole were filled into 6000 hard gelatin capsule.

### COMPARATIVE EXAMPLE B: Dipyridamole 200 mg extended release capsules having acacia present in the drug layer.

| **Ingredient** | **mg/Capsule** |
|---|---|
| Tartaric acid pellets | 200 |

| SEAL COATING: Stage 1 | |
|---|---|
| Hypromellose 15 cps | 26.7 |
| Talc | 13.3 |
| Isopropyl alcohol* | 600 |
| Methylene chloride* | 600 |

| SEAL COATING: Stage 2 | |
|---|---|
| Stearic acid | 16 |
| Isopropyl alcohol* | 425.7 |

| DRUG LAYERING | |
|---|---|
| Dipyridamole | 200 |
| Acacia | 10 |
| Hypromellose 5 cps | 11.3 |
| Isopropyl alcohol* | 221 |
| Methylene chloride* | 221 |

| MODIFIED RELEASE COATING | |
|---|---|
| Eudragit S100 | 12.4 |
| Hypromellose phthalate 55 | 12.4 |
| Hypromellose 5 cps | 7.5 |
| Dimethicone | 1.7 |
| Triacetin | 4.8 |
| Isopropyl alcohol* | 271 |
| Acetone* | 271 |

| | |
|---|---|
| * Evaporate during processing. | |

Manufacturing process:
1. Hypromellose and talc were dispersed in the isopropyl alcohol and methylene chloride mixture.
2. Tartaric acid pellets were coated with the dispersion of step 1 to a weight built up of 20% w/w in a fluid bed processor.
3. Stearic acid was dissolved in isopropyl alcohol, and the pellets of step 2 were coated with this solution to achieve a weight build up of 8 % w/w in a fluid bed processor.
4. Hypromellose and talc were dispersed in the isopropyl alcohol and methylene chloride mixture.
5. Dipyridamole and acacia were mixed.
6. Pellets of step 3 were coated with the mixture of step 5 by a powder layering technique using the binder dispersion of step 4.
7. The drug loaded pellets of step 6 were coated in a fluid bed processor with a modified release dispersion containing Eudragit, hypromellose phthalate, hypromellose, dimethicone and triacetin, dispersed in the mixture of isopropyl alcohol and acetone, to achieve a weight built up of 8 % w/w.
8. Modified release coated pellets of step 7 equivalent to 200 mg dipyridamole were filled into hard gelatin capsules.

### EXAMPLE 6: Stability study of dipyridamole 200 mg extended release capsules prepared in Example 1.

Storage conditions: 40°C and 75% relative humidity, for 1 month.
Stability packaging: 60 capsules filled into a sealed HDPE container (150 cc) with 2 molecular sieve desiccant pouches.

Twenty capsules from each of the initial and the stored capsules were analyzed by a high performance liquid chromatography technique, to determine impurities. The impurity contents are expressed below as percentages of the amount of dipyridamole present in a capsule.

| **Parameter (values in % w/w)** | **COMPARATIVE EXAMPLE A** | | **COMPARATIVE EXAMPLE B** | | **EXAMPLE 1** | |
|---|---|---|---|---|---|---|
| | **Initial** | **After Storage** | **Initial** | **After storage** | **Initial** | **After Storage** |
| Impurity A | 0.095 | 0.097 | 0.098 | 0.104 | 0.014 | 0.091 |
| Impurity B | ND* | 0.018 | ND* | ND* | ND* | ND* |
| Tartaric acid adduct | 1.711 | 7.998 | 0.13 | 0.826 | 0.02 | 0.386 |
| Total Impurities | 1.952 | 11.045 | 0.356 | 1.324 | 0.09 | 0.744 |

| | | | | | | |
|---|---|---|---|---|---|---|
| * Not detected. Limit of detection about 0.004 % w/w. | | | | | | |

## Claims

1. A pharmaceutical composition comprising pharmaceutically inert core particles having an intermediate coating, a dipyridamole-containing layer covering a coated core, and a drug release modifying layer covering a dipyridamole-containing layer.

2. The pharmaceutical composition of claim 1, wherein core particles comprise an organic acid.

3. The pharmaceutical composition of claim 1, wherein core particles comprise tartaric acid.

4. The pharmaceutical composition of any of claims 1-3, wherein an intermediate coating comprises more than one layer of coating.

5. The pharmaceutical composition of any preceding claim, wherein an intermediate coating comprises a hydrocolloid.

6. The pharmaceutical composition of any preceding claim, wherein an intermediate coating comprises a nonaqueous layer comprising a cellulose derivative, covered by a layer comprising a hydrocolloid.

7. The pharmaceutical composition of any preceding claim, wherein an intermediate coating comprises acacia gum.

8. The pharmaceutical composition of any preceding claim; comprising tartaric acid core particles having an intermediate coating comprising a hydrocolloid, a dipyridamole-containing layer covering a coated core, and a drug release modifying layer covering a dipyridamole-containing layer.

9. A process for preparing a pharmaceutical composition comprising:
a) applying an intermediate coating comprising one or more layers comprising the same or different pharmaceutically acceptable excipients or mixtures of excipients over pharmaceutically inert core particles;
b) applying a coating comprising dipyridamole and optionally one or more pharmaceutically acceptable excipients upon coated core particles; and
c) applying a coating comprising at least one drug release modifying substance, upon particles coated with dipyridamole.

10. The process of claim 9, wherein pharmaceutically inert core particles comprise tartaric acid.

11. The process of claims 9 or 10, wherein an intermediate coating comprises a hydrocolloid.
